# FASCICULE DE BREVET EUROPEEN

(11) **EP 1 187 919 B1**
(45) Date de publication et mention de la délivrance du brevet: **21.11.2007**
(21) Numéro de dépôt: 00940476.5
(22) Date de dépôt: 08.06.2000
(51) Int. Cl.: C12N 15/12, C12N 15/86, A61K 48/00

(54) **ADENOVIRUS RECOMBINANTS CODANT POUR LE TRANSPORTEUR SPECIFIQUE DE L'IODE (NIS)**
REKOMBINANTE, NATRIUM-JODID SYMPORTER-KODIERENDE ADENOVIREN
RECOMBINANT ADENOVIRUSES ENCODING THE SODIUM/IODIDE SYMPORTER (NIS)

(30) Priorité: 11.06.1999 FR 9907449
(43) Date de publication de la demande: 20.03.2002
(73) Titulaire: Aventis Pharma S.A., 92160 Antony (FR)
(72) Inventeur: PERRICAUDET, Michel, F-28320 Ecrosnes (FR); SCHLUMBERGER, Martin, F-92100 Boulogne Billancourt (FR); YEH, Patrice, F-91190 Gif sur Yvette (FR); BOLAND-AUGE, Anne, F-94240 L'Hay-les-Roses (FR)
(86) Numéro de dépôt international: PCT/FR2000/001594
(87) Numéro de publication internationale: WO 2000/076450

(56) Documents cités:
- WO-A-94/28152
- MANDELL ROBERT B ET AL: "Radioisotope concentrator gene therapy using the sodium/iodide symporter gene." CANCER RESEARCH FEB. 1, 1999, vol. 59, no. 3, 1 février 1999 (1999-02-01), pages 661-668, XP002134372 ISSN: 0008-5472 cité dans la demande
- MANDELL R ET AL: "Gene therapy of cancer by retroviral transfer and expression of the rat sodium/iodide symporter (NIS)." EIGHTY-EIGHTH ANNUAL MEETING OF THE AMERICAN ASSOCIATION FOR CANCER RESEARCH;SAN DIEGO, CALIFORNIA, USA; APRIL 12-16, 1997, vol. 38, 1997, page 381 XP002134373 Proceedings of the American Association for Cancer Research Annual Meeting 1997 ISSN: 0197-016X
- DATABASE BIOSIS [en ligne] BIOSCIENCES INFORMATION SERVICE, PHILADELPHIA, PA, US; mai 2000 (2000-05) CHO J -Y ET AL: "Expression and activity of human Na+/I- symporter in human glioma cells by adenovirus-mediated gene delivery." Database accession no. PREV200000254369 XP002155151 & GENE THERAPY, vol. 7, no. 9, mai 2000 (2000-05), pages 740-749, ISSN: 0969-7128
- KOVESDI I. ET AL: "Adenoviral vectors for gene transfer" CURRENT OPINION IN BIOTECHNOLOGY, vol. 8, no. 5, octobre 1997 (1997-10), pages 583-589,
- CHO J -Y; XING S; LIU X; BUCKWALTER T L F; HWA L; SFERRA T J; CHIU I-M; JHIANG S M: "Expression and activity of human Na+/I- symporter in human glioma cells by adenovirus-mediated gene delivery" GENE THERAPY, vol. 7, no. 9, 2 mai 2000 (2000-05-02), pages 740-749, XP001037550

## Description

La présente invention concerne le domaine de la thérapie génique et le traitement des tumeurs. L'invention concerne plus particulièrement l'introduction d'un gène codant pour le transporteur spécifique de l'iode (Na⁻/I⁻ Symporteur) NIS dans des cellules tumorales au moyen d'un vecteur adénoviral pour favoriser l'accumulation d'iode dans ces cellules. L'invention concerne également les adénovirus recombinants défectifs pour la réplication comprenant le gène *nis* et l'utilisation de ces vecteurs dans une méthode de traitement des cancers associant le transfert du gène *nis* dans des cellules tumorales et la radiothérapie métabolique par l'iode 131.

L'iode 131 est utilisé depuis plus de cinquante ans dans le traitement des cancers différenciés de la thyroïde. Son efficacité thérapeutique est liée à la dose de radiations délivrée au tissu tumoral. Par exemple dans le cas de métastases, une réponse tumorale après traitement par l'iode 131 est observée lorsque la dose délivrée au niveau du tissu tumoral est supérieure à 80 grays alors que le taux de réponse tumorale est faible ou nul pour des doses inférieures à 35 grays [Maxon , NEJM, 309 : 937-941, 1983]. On peut généralement estimer que les doses totales nécessaires pour traiter ou réduire le volume d'une tumeur varient entre 40 et 60 grays en fonction de leur radiosensibilité. Cette dose totale peut être délivrée lors de plusieurs traitement par l'iode 131, comme cela est réalisé lors du traitement des métastases fixantes de cancer thyroïdien différencié.

La dose de radiation délivrée au tissu tumoral dépend de deux facteurs biologiques : la demi-vie effective (T_{eff}) de l'iode 131 dans le tissu tumoral et la concentration radioactive.
- La demi-vie effective (T_{eff}) de l'iode 131 dans le tissu tumoral dépend de la demi-vie biologique (T_{biol}) et de la demi-vie physique (T_{phys}) selon la relation 1/T_{eff} = 1/T_{biol}+1/T_{phys}. La demi-vie biologique (T_{biol}) de l'iode 131 dans les cellules fixantes dépend de leur capacité d'organification de l'iode. En l'absence d'organification, comme par exemple dans les cellules non thyroïdiennes, la demi-vie biologique est brève, de quelques heures à quelques dizaines d'heures. La demie-vie physique (T_{phys}) de l'iode 131 est de 8.02 jours. Ainsi, au mieux, on peut estimer que la demi-vie effective de l'iode 131 dans un tissu tumoral est de quelques heures à quelques dizaines d'heures.
- La concentration radioactive est le rapport entre la fixation globale de l'iode 131 par le tissu tumoral et la masse de ce tissu.

A titre d'exemple, pour une fixation d'iode de 0.1 % de l'activité administrée pour 1 g de tissu et une demie-vie effective de 1.5 jours, on peut estimer que l'administration de 3,7 gigabecquerel (ou 100 mCi) délivre une une dose de 30 grays au tissu tumoral.

La fixation globale d'iode 131 est un paramètre qui dépend d'une part, de l'activité de l'iode 131 administrée au patient et d'autre part, des capacités du tissu tumoral à concentrer l'iode 131.
- L'activité maximale d'iode 131 pouvant être administrée à un patient est limitée par la toxicité de l'iode 131 résultant des doses d'irradiation délivrées aux tissus sain et notamment à la moelle osseuse. Ainsi en cas de cancer de la thyroïde, les activités thérapeutiques administrées, alors que les patients sont en état d'hypothyroïdie, sont en général comprises entre 3.7 et 10 GBq (100 à 300 mCi). Les activités maximales pouvant être administrées à des patients euthyroïdiens sont plus élevées car la rétention de l'iode est alors deux fois inférieure à celle observée en cas d'hypothyroïdie), elles atteignent des valeurs comprises entre 18,5 et 22.2 GBq (500 à 600 mCi).
- La capacité du tissu tumoral à concentrer l'iode 131, pour une activité déterminée administrée au patient, dépend du niveau d'expression du symporteur de l'iode et de son activité biologique. Ceci a été démontré par l'étude des tissus thyroïdiens humains normaux et pathologiques et par des études *in vitro* de l'activité du NIS. L'activité spécifique de l'iode 131 étant élevée, il n'a pas été observé de phénomène de saturation avec augmentation de l'activité d'iode 131 administrée.

En raison de la limitation de l'activité maximale d'iode 131 pouvant être administrée au patient, il serait souhaitable de pouvoir accroître la capacité du tissu tumoral à concentrer l'iode 131 pour augmenter la fixation globale de l'iode 131.

Il est également déterminant que l'augmentation de la fixation de l'iode se fasse de manière homogène au niveau du tissu tumoral. En effet, la fixation de l'iode dans un tissu fixant peut être très hétérogène d'une cellule à l'autre, voire d'une zone tumorale à une autre. La dose d'irradiation au niveau du tissu tumoral est délivrée essentiellement par l'émission β de l'iode 131. Or son parcours dans les tissus biologiques est au maximum de à 3 mm. De plus, autour d'une source ponctuelle, la dose d'irradiation diminue de manière exponentielle avec la distance. Ces éléments renforcent l'importance d'atteindre une forte concentration radioactive au niveau des zones tumorales fixantes et d'autre part, la nécessité d'obtenir une fixation d'iode aussi homogène que possible.

Il faut noter que la fixation de l'iode 131 par la thyroïde humaine , et donc son irradiation, peut être facilement supprimée (fixation d'iode 131 non mesurable) par l'administration de L-triiodothyronine 1 µg/kg/jour divisée en trois prises quotidiennes pendant trois semaines.

Le transporteur NIS est responsable de la concentration de l'iode circulant (sous forme d'iodure I) par les thyréocytes [pour une revue voir P. Thomopoulos , Médecine et Science, vol 4(10), p. 825-828]. La concentration de l'iode dans ces cellules présente les caractéristiques suivantes : l'iode est concentré d'un facteur 30 à 40 fois contre un gradient électrochimique; il requiert la présence de sodium (Na⁻) ; il s'agit d'un transport actif , il est inhibé de manière compétitive par certains anions, tels le thiocyanate, le perchlorate, le pertechnétate. La concentration de l'iode par les thyréocytes est suivi de l'organification de l'iode et la synthèse d'iodotyrosines et d'hormones thyroïdiennes (thyroxine T4 et triiodothyronine T3).

Les gènes codant pour le transporteur murin [Dai et al. Nature 379 : 458 - 460 (1996)] et le transporteur humain [Smanik P.A. et al. Biochem. Biophys. Res. Commun. 226 : 339 - 345 (1996)] de l'iode NIS ont été isolés, ils sont identiques à 84 %. Le gène est localisé sur le chromosome 19p dans l'espèce humaine. Il comporte 15 exons séparés par 14 introns. Sa transcription donne naissance à deux formes par épissage alternatif, dont la forme longue prédomine dans la thyroïde. La protéine a un poids moléculaire de 55 kDa, atteignant 80 kDa après glycosylation. Elle est localisée dans la membrane latérobasale des thyréocytes. Ses extrémités aminoterminale et carboxyterminale sont intracellulaires tandis que le reste de la chaîne peptidique comporte 13 segments transmembranaires réunis par des boucles intracellulaires et extracellulaires [Levy et al., 1998. J. Biol. Chem. 273 : 22657-22663]. L'introduction du gène codant pour NIS dans des cellules non thyroïdiennes leur confère la capacité à capter l'iode, avec les mêmes propriétés que les thyréocytes, en particulier la présence obligatoire de sodium (Na+) et l'inhibition par les anions perchlorates.

La transcription du gène *nis* est activée dans les thyréocytes par la TSH. Cet effet est médié par l'AMP cyclique. La demi-vie de la protéine, dans les thyréocytes murins est de 4 jours [Paire A. et al. J. Biol. Chem. 272 18245 - 18249 (1997)]. Dans les tissus extrathyroïdiens, l'activité du gène est plus faible que celle de la thyroïde à l'état basal. Ceci est vraisemblablement dù à la stimulation de la transcription du gène *nis* dans les thyréocytes, par le facteur spécifique de transcription TTF-1 (thyroid transcription factor 1), qui peut se lier au promoteur du gène *nis* thyroïdien, mais non à celui des autres tissus [Endo T. et al. Mol. Endocrinol. 11: 1747 - 1755 (1997)]. En dehors de la thyroïde, certains tissus sont capables de capter et de concentrer l'iode notamment les glandes salivaires et la muqueuse gastrique.

Une étude récente rapporte le transfert du gène *nis* au moyen d'un vecteur rétroviral dans des cellules tumorales humaines ou murines [Mandell et al. Cancer Research 59: 661-668 (1999)]. Ces résultats sont intéressants en ce qu'ils montrent qu'il est possible d'exprimer le gène *nis* dans des cellules non thyroïdiennes et d'observer une concentration de l'iode dans des cellules qui n'accumulent pas cet élément naturellement. Cependant un certain nombre de facteurs limitants restent à surmonter afin d'atteindre un niveau de fixation de l'iode suffisant pour envisager une application thérapeutique.

Un premier facteur est le faible niveau d'expression du gène *nis* au niveau des cellules tumorales. Par exemple, dans l'étude précitée, les résultats obtenus *in vitro* montrent que la concentration d'iode atteinte dans les cellules tumorales non thyroïdiennes reste environ deux fois inférieure à la concentration d'iode accumulée dans les cellules thyroïdiennes.

De plus, compte tenu des perturbations importantes que présentent les cellules tumorales au niveau membranaire, il apparaît difficile d'assurer l'intégration fonctionnelle d'un transporteur avec une efficacité comparable à celle des cellules thyroïdiennes non tumorales.

Un autre facteur limitant est l'absence d'organification de l'iode au niveau des cellules non thyroïdiennes, or cette organification de l'iode est un élément nécessaire au maintient de l'iode dans les cellules. Ainsi dans l'étude rapportée ci-dessus, il est observé un efflux très rapide de l'iode accumulé *in vitro* (entre 30 et 60 min) pour les cellules non thyroïdiennes.

Enfin compte tenu de l'hétérogénéité des cellules tumorales et des modifications importantes de ces cellules au niveau membranaire, il semble difficile de pouvoir atteindre une répartition homogène du transporteur NIS au niveau des membranes des cellules tumorales.

Ainsi aucune approche décrite à ce jour n'a encore permis d'atteindre une accumulation de l'iode au niveau de tumeur non thyroïdienne avec un niveau d'expression suffisant pour envisager l'utilisation de l'iode 13 1 pour le traitement des tumeurs non thyroïdiennes.

La présente invention présente une méthode améliorée de traitement des tumeurs combinant la thérapie génique et la radiothérapie à l'iode 131

La présente invention décrit en particulier une méthode permettant d'augmenter l'efficacité de la fixation de l'iode au niveau des tumeurs non thyroidiennes et permettant ainsi d'appliquer aux tumeurs non thyroïdiennes, les principes de radiothérapie développés avec succès pour le traitement des cancers thyroïdiens.

La présente invention résulte de la mise en évidence que le transfert du gène codant pour le transporteur spécifique de l'iode (Na⁻/I⁻ Symporteur) NIS au moyen d'un adénovirus recombinant défectif permet d'atteindre une accumulation d'iode très importante au niveau de cellules tumorales non thyroïdiennes. Dans un mode de réalisation spécifique, l'apport d'un adénovirus défectif exprimant le gène *nis* permet d'accumuler l'iode à des concentrations environ cinq fois supérieures à celles observées dans les cellules thyroïdiennes. Cette capacité surprenante des cellules tumorales non thyroïdiennes à accumuler l'iode 125 permet d'envisager pour ce type de tumeurs une nouvelle approche thérapeutique basée sur la radiothérapie métabolique par l'iode 131; approche jusqu'alors réservée au traitement de certaines tumeurs thyroïdiennes.

L'intérêt majeur de la radiothérapie métabolique est de délivrer des doses d'irradiation importantes aux tissus fixants l'isotope radioactif, sans irradier significativement les tissus environnants.

La découverte qu'il est effectivement possible de concentrer de manière importante l'iode dans des cellules tumorales qui sont normalement incapables d'accumuler cet élément permet d'envisager l'application de cette méthode dans de nombreuses indications pour le traitement des tumeurs d'origine non thyroïdienne. Deux types d'indications sont plus particulièrement envisagées : les tumeurs difficilement accessibles à la radiothérapie externe en raison de leur localisation, à titre d'exemple on peut citer les cancers de la prostate inopérables ou les tumeurs intracérébrales ; les tumeurs ayant déjà été irradiées et pour lesquelles un complément de radiothérapie externe est impossible, car les doses maximales ont déjà été délivrées : ceci concerne tous les cancers survenant ou rechutant en zones irradiées. Cette méthode est également applicable au traitement des tumeurs thyroïdiennes ayant perdu la capacité de capter l'iode et ne répondant pas à la thérapie métabolique.

II est également envisageable de combiner la radiothérapie métabolique et la radiothérapie externe comme cela est réalisé en cas de métastases de cancer différencié de la thyroïde. Cette combinaison permet d'augmenter l'efficacité thérapeutique de la radiothérapie, sans en augmenter la toxicité. Cette combinaison paraît particulièrement intéressante pour les tumeurs peu sensibles à la radiothérapie. Par ailleurs, elle permet d'envisager l'application de cette technique à des tumeurs qui ne seraient pas accessibles à la radiothérapie métabolique seule, en raison de leur taille trop importante.

Un premier objet de l'invention réside donc dans un adénovirus recombinant défectif comprenant au moins une séquence d'ADN hétérologue codant pour le transporteur spécifique de l'iode (Na⁺/I⁻ Symporteur) NIS, ou un de ses dérivés.

Au sens de la présente invention on entend par "dérivé" du transporteur spécifique de l'iode (Na⁻/I' Symporteur) NIS tout analogue, fragment ou forme mutée qui est dérivé du polypeptide NIS et qui conserve une activité de transport spécifique de l'iode. Différents dérivés peuvent exister à l'état naturel. Ces dérivés peuvent être des variations alléliques caractérisées par des différences dans la séquence nucléotidique des gènes de structure codant pour le NIS ou peuvent résulter d'un épissage différentiel ou de modifications post-traductionnelle. Ces dérivés peuvent être obtenus par substitution, délétion, addition et/ou modification d'un ou plusieurs résidus acides aminés. Ces modifications peuvent être réalisées par toutes techniques connues de l'homme du métier. Ces dérivés sont notamment des molécules ayant une plus grande affinité pour leurs substrats, des séquences permettant une expression améliorée *in vivo*, des molécules présentant une plus grande résistance aux protéases, des molécules ayant une efficacité biologique plus grande ou des effets secondaires moindres, ou éventuellement de nouvelles propriétés biologiques. D'autres dérivés utilisables dans le cadre de l'invention sont notamment les molécules dans lesquelles un ou plusieurs résidus ont été substitués, les dérivés obtenus par délétion de régions n'intervenant pas ou peu dans l'interaction avec les sites de liaison considérés ou exprimant une activité indésirable, et les dérivés comportant par rapport à la séquence native des résidus supplémentaires, tels que par exemple un signal de sécrétion et/ou un peptide de jonction.

Le transporteur spécifique de l'iode (Na⁻/I⁻ Symporteur) NIS ou son dérivé, produit dans le cadre de la présente invention peut être un ADNc, un ADN génomique (ADNg), ou une construction hybride consistant par exemple en un ADNc dans lequel seraient insérés un ou plusieurs introns. Il peut également s'agir de séquences synthétiques ou semi-synthétiques. De manière avantageuse, on utilise un ADNc ou un ADNg. En particulier, l'utilisation d'un ADNg peut permettre une meilleure expression dans les cellules humaines.

Selon un premier mode de réalisation, il s'agit d'une séquence d'ADNc codant pour le transporteur spécifique de l'iode (Na⁺ /I⁻ Symporteur) NIS d'origine murine Selon un mode préféré de l'invention, il s'agit d'une séquence d'ADNc codant pour le transporteur spécifique de l'iode (Na⁺ /I⁻ Symporteur) NIS d'origine humaine.

Les adénovirus utilisés dans le cadre de la présente invention sont des adénovirus recombinants, c'est à dire comprenant une séquence d'ADN hétérologue. Avantageusement, il s'agit d'adénovirus recombinants défectifs, c'est à dire incapables de réplication autonome dans les cellules cibles.

Pour la construction des adénovirus selon l'invention, différents sérotypes peuvent être utilisés. Il existe en effet de nombreux sérotypes, dont la structure et les propriétés varient quelque peu, mais qui présentent une organisation génétique comparable. Plus particulièrement, les adénovirus recombinants peuvent être d'origine humaine ou animale. Concernant les adénovirus d'origine humaine, on peut citer préférentiellement ceux classés dans le groupe C, en particulier les adénovirus de type 2 (Ad2), 5 (Ad5), 7 (Ad7) ou 12 (Ad12). Parmi les différents adénovirus d'origine animale, on peut citer préférentiellement les adénovirus d'origine canine, et notamment toutes les souches des adénovirus CAV2 [souche manhattan ou A26/61 (ATCC VR-800) par exemple]. D'autres adénovirus d'origine animale sont cités notamment dans la demande WO94/26914.

Le génome des adénovirus comprend notamment une séquence inversée répétée (ITR) à chaque extrémité, une séquence d'encapsidation (Psi), des gènes précoces et des gènes tardifs. Les principaux gènes précoces sont contenus dans les régions E1, E2, E3 et E4. Parmi ceux-ci, les gènes contenus dans la région E1 notamment sont nécessaires à la propagation virale. Les principaux gènes tardifs sont contenus dans les régions L1 à L5. Le génome de l'adénovirus Ad5 a été entièrement séquencé et est accessible sur base de données (voir notamment Genbank M73260). De même des parties, voire la totalité d'autres génomes adénoviraux (Ad2, Ad7, Ad 12, etc) ont également été séquencées.

Comme indiqué ci-avant, les adénovirus selon l'invention sont défectifs et sont donc incapables de se répliquer de façon autonome dans la cellule cible. A cet effet, différentes constructions dérivées des adénovirus ont été préparées, incorporant différents gènes thérapeutiques. Dans chacune de ces constructions, l'adénovirus a été modifié de manière à le rendre incapable de réplication dans la cellule infectée. Ainsi, les constructions décrites dans l'art antérieur sont des adénovirus délétés de la région E1, essentielle à la réplication virale, au niveau de laquelle sont insérées les séquences d'ADN hétérologue (Levrero et al., Gene 101 (1991) 195 ; Gosh-Choudhury et al., Gene 50 (1986) 161). Par ailleurs, pour améliorer les propriétés du vecteur, il a été proposé de créer d'autres délétions ou modifications dans le génome de l'adénovirus. Ainsi, une mutation ponctuelle thermosensible a été introduite dans le mutant ts 125, permettant d'inactiver la protéine de 72kDa de liaison à l'ADN (DBP) (Van der Vliet et al., J. Virol., 1975, 15(2) 348-354). D'autres vecteurs comprennent une délétion d'une autre région essentielle à la réplication et/ou à la propagation virale, la région E4. La région E4 est en effet impliquée dans la régulation de l'expression des gènes tardifs, dans la stabilité des ARN nucléaires tardifs, dans l'extinction de l'expression des protéines de la cellule hôte et dans l'efficacité de la réplication de l'ADN viral. Des vecteurs adénoviraux dans lesquels les régions E1 et E4 sont délétées possèdent donc un bruit de fond de transcription et une expression de gènes viraux très réduits. De tels vecteurs ont été décrits pas exemple dans les demandes WO94/28152, WO95/02697, WO96/22378. En outre des vecteurs portant une modification au niveau du gène IVa2 ont également été décrits (WO96/10088).

Dans un mode préféré de mise en oeuvre de l'invention, l'adénovirus recombinant est un adénovirus humain du groupe C. De manière plus préférentielle, il s'agit d'un adénovirus Ad2 ou Ad5.

Avantageusement, l'adénovirus recombinant utilisé dans le cadre de l'invention comprend une délétion dans la région E1 de son génome. Encore plus particulièrement, il comprend une délétion des régions E1a et E1b. A titre d'exemple, on peut citer des délétions affectant les nucléotides 454-3328 , 386-3446 , 459-3510 ou 357-4020 (par référence au génome de l'Ad5).

Selon une autre variante, l'adénovirus recombinant utilisé dans le cadre de l'invention est défectif pour tout ou partie des régions E1 et E3 au moins.

Selon une autre variante, l'adénovirus recombinant utilisé dans le cadre de l'invention comprend outre une délétion dans la région E1, une délétion affectant tout ou partie de la région E4 de son génome. Plus particulièrement, la délétion dans la région E4 affecte l'ensemble des phases ouvertes. On peut citer à titre d'exemple précis les délétions 33466-35535 ou 33093-35535 ou une partie seulement de la région E4 (ORF6 ou ORF3 par exemple), comme décrit dans les demandes WO95/02697 et WO96/22378.

Il peut s'agir par exemple d'adénovirus recombinants dits de 3^{ème} génération, c'est à dire défectifs pour les régions E1 et E4, en tout ou partie, et éventuellement pour la région E3. Des variantes particulières de l'invention sont constituées par l'utilisation d'adénovirus portant des délétions affectant tout ou une partie des régions fonctionnelles suivantes :
- E1,E4 et E3,
- E1, E4 et E2,
- E1, E4, E2 et E3,
- les régions ci-dessus ainsi que tout ou partie des gènes codant pour les fonctions tardives de l'adénovirus (L1 à L5), ou encore,
- l'ensemble des régions virales codantes.

Concernant les adénovirus dépourvus en plus des fonctions tardives (vecteur "minimum") ou de l'ensemble des régions codantes (vecteur "gutless"), leur construction a été décrite par exemple par Parks et al. PNAS 93 (1996) p. 13565 et Lieber et al., J. Virol. 70 (1996) p. 8944.

Il peut s'agir également de vecteurs adénoviraux hybrides tels que ceux décrits dans la demande WO99/60144 et dans lesquels l'adénovirus est utilisé comme vecteur d'un autre virus et notamment comme vecteur de rétrovirus.

La cassette d'expression contenant l'acide nucléique codant pour le transporteur de l'iode (NIS) peut être insérée en différents sites du génome recombinant. Elle peut être insérée au niveau de la région E1, E3 ou E4, en remplacement des séquences délétées ou en surplus. Elle peut également être insérée en tout autre site, en dehors des séquences nécessaires en cis à la production des virus (séquences ITR et séquence d'encapsidation).

Au sens de la présente invention on entend par "cassette d'expression" d'un acide nucléique, un fragment d'ADN qui peut être inséré dans un vecteur à des sites de restrictions spécifiques ; le fragment d'ADN comprend, outre la séquence nucléotidique codant pour un ARN ou un polypeptide d'intérêt, les séquences nécessaires à l'expression (enhanceur(s), promoteur(s), séquence de polyadénylation ...) de ladite séquence d'intérêt. Le fragment d'ADN et les sites de restriction sont conçus pour assurer une insertion dudit fragment dans un cadre de lecture approprié pour la transcription et/ou la traduction.

Les adénovirus recombinants sont produits dans une lignée d'encapsidation, c'est-à-dire une lignée de cellules capables de complémenter en trans une ou plusieurs des fonctions déficientes dans le génome adénoviral recombinant. Parmi les lignées d'encapsidation connues par l'homme du métier, on peut citer par exemple la lignée 293 dans laquelle une partie du génome de l'adénovirus a été intégrée. Plus précisément, la lignée 293 est une lignée de cellules embryonnaires humaines de rein contenant l'extrémité gauche (environ 11-12 %) du génome de l'adénovirus sérotype 5 (Ad5), comprenant l'ITR gauche, la région d'encapsidation, la région E1, incluant E1 a et E1b, la région codant pour la protéine pIX et une partie de la région codant pour la protéine pIVa2. Cette lignée est capable de trans-complémenter des adénovirus recombinants défectifs pour la région E1, c'est-à-dire dépourvus de tout ou partie de la région E1, et de produire des stocks viraux ayant des titres élevés. Cette lignée est également capable de produire, à température permissive (32°C), des stocks de virus comportant en outre la mutation E2 thermosensible. D'autres lignées cellulaires capables de complémenter la région E ont été décrites, basées notamment sur des cellules de carcinome de poumon humain A549 (WO94/28152) ou sur des rétinoblastes humains (Hum. Gen. Ther. (1996) 215). Par ailleurs, des lignées capables de trans-complémenter plusieurs fonctions de l'adénovirus ont également été décrites. En particulier, on peut citer des lignées complémentant les régions E et E4 (Yeh et al., J. Virol. Vol. 70 (1996) pp 559-565; Cancer Gen. Ther. 2 (1995) 322 ; Krougliak et al., Hum. Gen. Ther. 6 (1995) 1575) et des lignées complémentant les régions E1 et E2 (WO94/28152, WO95/02697, WO95/27071) ou des lignées dérivées de celles-ci utilisables pour produire des adénovirus minimum, en particulier parce qu'elles expriment en outre une activité de recombinase site spécifique impliquée dans la construction de tels virus.

Les adénovirus recombinants peuvent également être modifiés dans la structure de la capside pour augmenter l'efficacité d'infection au niveau de la tumeur. Par exemple la capside peut contenir un ligand de uPAR ou un motif RGD qui permet le ciblage de l'adénovirus vers les cellules tumorales, de tels vecteurs et des séquences de ciblage ont été décrits notamment dans la demande WO 00/12738. Les séquences de ciblage peuvent être insérées dans la protéine de l'hexon ou dans la protéine de la fibre. De préférence les séquences de ciblages sont insérées au niveau de délétion de la protéine de la fibre ou de l'hexon. La séquence polypeptidique de l'hexon est avantageusement délétée de 13 amino acides correspondant aux positions 279 à 292 de la séquence polypeptidique de l'hexon de Ad 5. La séquence polypeptidique de la fibre est avantageusement délétée de 11 amino acides correspondant aux positions 539 à 547 de la séquence polypeptidique de la fibre (HI Loop) de Ad 5.

Les adénovirus recombinants sont habituellement produits par introduction de l'ADN viral dans la lignée d'encapsidation, suivie d'une lyse des cellules après environ 2 ou 3 jours (la cinétique du cycle adénoviral étant de 24 à 36 heures). Pour la mise en oeuvre du procédé, l'ADN viral introduit peut être le génome viral recombinant complet, éventuellement construit dans une bacterie (WO96/25506) ou dans une levure (WO95/03400), infecté dans les cellules. Il peut également s'agir d'un virus recombinant utilisé pour infecter la lignée d'encapsidation. L'ADN viral peut aussi être introduit sous forme de fragments portant chacun une partie du génome viral recombinant et une zone d'homologie permettant, après introduction dans la cellule d'encapsidation, de reconstituer le génome viral recombinant par recombinaison homologue entre les différents fragments.

Après la lyse des cellules, les particules virales recombinantes peuvent être isolées par toute technique connue telle que la centrifugation en gradient de chlorure de césium ou la chromatographie. Une méthode alternative a été décrite dans la demande WO98/00528.

*Séquences de régulation de l'expression.* Le gène codant pour le transporteur spécifique de l'iode (Na⁺/I⁻ Symporteur, NIS) peut être placé sous le contrôle de toute séquence régulatrice de l'expression telle que par exemple un promoteur ou un promoteur /enhancer, fonctionnelle et permettant l'expression dans les cellules hôtes tumorales.

Les séquences régulatrices de l'expression peuvent comprendre, outre la région promotrice, une région située en 3' du gène codant pour le transporteur spécifique de l'iode et qui fournit un signal de fin de transcription et un site de polyadénylation. L'ensemble de ces éléments constitue une cassette d'expression.

Le promoteur peut être constitutif ou régulable (inductible). Il peut s'agir du propre promoteur du gène. Il peut également s'agir de séquences d'origine différente (responsables de l'expression d'autres protéines, ou même d'un promoteur synthétique). Notamment, il peut s'agir de séquences promotrices de gènes eucaryotes ou viraux. Par exemple, il peut s'agir de séquences promotrices issues du génome de la cellule que l'on désire transfecter. De même, il peut s'agir de séquences promotrices issues du génome d'un virus, y compris du virus utilisé. A cet égard, on peut citer par exemple les promoteurs E1A, MLP, CMV, LTR-RSV, MT-1, SV40 etc.

En outre, ces séquences d'expression peuvent être modifiées par addition de séquences d'activation, de régulation, permettant une expression tissu-spécifique ou prédominante dans certains tissus (promoteur énolase, GFAP, etc.). Il peut en effet être particulièrement intéressant d'utiliser des signaux d'expression actifs spécifiquement ou majoritairement dans les cellules tumorales, de manière à ce que le gène thérapeutique ne soit exprimé et ne produise son effet que lorsque le virus a effectivement infecté une cellule tumorale. Le caractère spécifique ou majoritaire de l'expression signifie que l'activité du promoteur est significativement très supérieure dans les cellules tumorales. Bien qu'une expression non-spécifique puisse exister dans d'autres cellules, le niveau d'activité correspondant reste généralement très faible (négligeable) par rapport à celui observé dans les cellules tumorales, généralement inférieur d'un facteur 10 au moins.

Parmi les promoteurs utilisables dans le cadre de l'invention on peut citer les promoteurs ubiquitaires (HPRT, vimentine, α-actine, tubuline, etc.), les promoteurs des filaments intermédiaires (desmine, neurofilaments, kératine, GFAP), les promoteurs de gènes d'intérêt thérapeutique (MDR, CFTR, facteur VIII etc.), les promoteurs spécifiques de tissus (promoteurs des gènes de la desmine, des myosines, de la créatine kinase, de la phophoglycérate kinase), les promoteurs plus spécifiquement actifs dans les cellules en croissance ou encore les promoteurs répondant à un stimulus tels que des promoteurs répondant aux hormones naturelles (récepteurs des hormones stéroïdes, récepteur de l'acide rétinoïque, etc.) ou un promoteur régulé par les antibiotiques (tétracycline, rapamycine, etc) ou d'autres promoteurs répondant à d'autres molécules d'origine naturelle ou synthétique ou de séquences promotrices issues du génome d'un virus comme l'enhancer/promoteur du cytomégalovirus CMV , le promoteur LTR du rétrovirus, le promoteur SV40, le promoteur du gène E1A, le promoteur MLP. Les promoteurs régulables par la tétracycline et le promoteur CMV ont été décrits dans WO96/01313, US 5,168,062 et US 5,385,839.

Parmi les promoteurs utilisables pour la mise en oeuvre de l'invention on peut citer notamment le promoteur du cytomegalovirus (CMV), la région promotrice early du SV40 (Benoist and Chambon, 1981, Nature 290:304-310), le promoteur compris dans la région 3' LTR du virus du sarcome de Rous (Yamamoto, et al., 1980, Cell 22:787-797), le promoteur de la thymidine kinase du virus de l'herpes (Wagner et al., 1981, Proc. Natl. Acad. Sci. U.S.A. 78:1441-1445), les séquences régulatrices du gène de la métallothioneine (Brinster et al., 1982, Nature 296:39-42); les promoteurs d'origine procaryotes comme le promoteur de la β-lactamase (Villa-Kamaroff, et al., 1978, Proc. Natl. Acad. Sci. U.S.A. 75:3727-3731), ou le promoteur *tac* (DeBoer, et al., 1983, Proc. Natl. Acad. Sci. U.S.A. 80:21-25); voir également "Useful proteins from recombinant bacteria" in Scientific American, 1980, 242:74-94; les promoteurs de levures tels que les promoteurs Gal4, ADC (alcool déshydrogénase), PGK (phosphoglycérol kinase), phosphatase alkaline ; et les séquences de régulation transcriptionnelle d'origine animale qui présentent une spécificité de tissus et qui sont utilisés pour les animaux transgéniques : les séquences de régulation du gène de l'élastase 1 qui sont actives dans les cellules des acini du pancréas (Swift , 1984, Cell 38:639-646; Ornitz et al., 1986, Cold Spring Harbor Symp. Quant. Biol. 50:399-409; MacDonald, 1987, Hepatology 7:425-515) ; les séquences de régulation du gène l'insuline qui sont actives dans les cellules beta du pancréas (Hanahan, 1985, Nature 315:115-122), les séquences de régulation de l'expression des immunoglobulines qui sont actives dans les cellules lymphoïdes (Grosschedl et al., 1984, Cell 38:647-658; Adames et al., 1985, Nature 318:533-538; Alexander et al., 1987, Mol. Cell. Biol. 7:1436-1444), la séquence de régulation du virus des tumeurs mammaires de souris qui est active dans les cellules des testicules, du sein, les lymphocytes et les mastocytes (Leder et al., 1986, Cell 45:485-495), la séquence de régulation du gène PSA qui est active dans les tumeurs prostatiques, la séquence de régulation du gène de l'albumine qui est active dans le foie (Pinkert et al., 1987, Genes and Devel. 1:268-276), la séquence de régulation du gène de l'alpha-foetoprotéine qui est active dans le foie (Krumlauf et al., 1985, Mol. Cell. Biol. 5:1639-1648; Hammer et al., 1987, Science 235:53-58), la séquence de régulation du gène de l'alpha 1-antitrypsine qui est active dans le foie (Kelsey et al., 1987, Genes and Devel. 1:161-171), la séquence de régulation du gène de la β-globine qui est active dans les cellules myéloides (Mogram et al., 1985, Nature 315:338-340; Kollias et al., 1986, Cell 46:89-94), la séquence de régulation du gène de la myéline basique qui est active dans les oligodendrocytes dans le cerveau (Readhead et al., 1987, Cell 48:703-712),), la séquence de régulation du gène de la chaîne légère 2 de la myosine qui est active dans le muscle squelettique (Sani, 1985, Nature 314:283-286), et la séquence de régulation du gène de l'hormone de libération des gonadotrophines qui est active au niveau de l'hypothalamus (Mason et al., 1986, Science 234:1372-1378).

Dans un mode particulier de réalisation de l'invention, on utilise un adénovirus recombinant défectif comprenant un gène codant pour le transporteur spécifique de l'iode (Na⁺/I⁻ Symporteur) NIS sous le contrôle d'un promoteur viral, choisi de préférence parmi le LTR-RSV ou le promoteur précoce du CMV.

Il est également possible d'associer l'introduction du gène NIS avec le vecteur selon l'invention à l'introduction d'un système biologique permettant l'augmentation de la demi-vie biologique de l'iode 131. Il est ainsi possible de délivrer dans les cellules tumorales de manière simultanée ou successive le gène NIS et un gène ou plusieurs gènes codant pour des polypeptides impliqués dans un système d'organification de l'iode. On peut citer par exemple les gènes codant pour des polypeptides impliqués dans un système peroxydasique tel que le gène codant pour la glucose oxidase.

On peut envisager de délivrer dans les cellules tumorales le gène NIS et un gène ou plusieurs gènes codant pour des polypeptides impliqués dans un système d'organification de l'iode au moyen d'un vecteur unique exprimant plusieurs transgènes ou par co-infection de deux vecteurs, un vecteur adénoviral exprimant le gène NIS et un autre vecteur, viral ou plasmidique, exprimant un ou plusieurs gènes codant pour des polypeptides impliqués dans un système d'organification de l'iode.

II est en outre possible d'associer l'introduction du gène NIS avec le vecteur selon l'invention à l'administration d'un système permettant un ralentissement de l'efflux de l'iode par un agent pharmacologique (par exemple, un sel de lithium).

Selon une autre variante, il est également envisageable d'associer l'introduction du gène NIS avec d'autres gènes d'intérêt thérapeutique pour le traitement des cancers. Il peut s'agir d'un gène suicide tel que le gène de la thymidine kinase de l'Herpès ou le gène de la cytosine déaminase. Il peut s'agir de gènes codant pour des protéines induisant l'apoptose comme p53, Bax, BcIX-s, Bad ou tout autre antagoniste de Bcl2 et de BcIX-1. Il peut s'agir de gènes codant pour des variants de ces protéines présentant des propriétés améliorées comme un variant de p53 (CTS-1 , WO97/04092). Il peut s'agir également de gènes codant pour des facteurs anti-angiogéniques ou angiostatiques tels que notamment le ligand de Tie-1 et de Tie-2, l'angiostatine, l'endostatine, le facteur ATF, les dérivés du plasminogène, l'endothéline, les thrombospondines 1 et 2, le PF-4, l'interféron α ou β, l'interleukine 12, le TNFα, le récepteur de l'urokinase, flt 1, KDR, PAI 1, PAI2, TIMP1, le fragment prolactine. Il peut s'agir également de gènes codant pour des protéines capables d'induire une immunité antitumorale ou stimuler la réponse immunitaire (IL2, GM-CSF, IL12, etc.). Parmi les gènes codant pour des protéines d'intérêt thérapeutique dans le traitement des cancers, il est également important de souligner les anticorps, les fragments variables d'anticorps simple chaîne (ScFv) ou tout autre fragment d'anticorps possédant des capacités de reconnaissance pour une utilisation en immunothérapie pour le traitement des tumeurs : anticorps anti-RAS (WO9429446).

De manière avantageuse, il peut s'agir d'un gène dont l'expression entraîne une radiosensibilisation des cellules tumorale comme le gène p53.

L'administration de ces gènes ou de la combinaison de deux ou plusieurs de ces gènes d'intérêt thérapeutique précités peut être réalisée par introduction d'un ou plusieurs de ces gènes dans le vecteur adénoviral comprenant le gène codant pour le transporteur NIS ou dans un vecteur séparé de nature virale ou non virale.

Selon une autre variante, le vecteur selon l'invention peut être administré en combinaison avec un agent anticancéreux tel que notamment, le taxol, le taxotère et autre taxoïdes [tels que décrits notamment dans U.S. 4,857,653 ; US 4,814,470 ; US 4,924,011; US 5,290,957 ; US 5,292,921 ; US 5,438,072 ; US 5,587,493 : EP 0 253 738 ; et WO91/17976 . WO93/00928, WO93/00929 , et WO9601815], ou d'autres agents thérapeutiques anticancéreux tels que le cis-platine et les dérivés du platine, l'étoposide and etoposide phosphate, bléomycine, mitomycine C, CCNU, doxorubicine, daunorubicine, idarubicine, ifosfamide, etc.

L'invention concerne également une composition pharmaceutique comprenant un vecteur adénoviral tel que décrit ci-avant et un véhicule physiologiquement acceptable. Les compositions pharmaceutiques de l'invention sont de préférence sous forme injectable et peuvent être formulées en vue d'une administration intratumorale ou par voie orale, parentérale, intranasale, intraartérielle, intraveineuse, intratrachéale etc.

Préférentiellement, la composition pharmaceutique contient des véhicules pharmaceutiquement acceptables pour une formulation destinée à être administrée par voie intratumorale.

Les compositions selon l'invention peuvent comprendre des doses variables d'adénovirus recombinants, aisément adaptables par l'homme du métier en fonction des applications envisagées et de différents paramètres, et notamment en fonction du mode d'administration utilisé ou encore de la durée de l'expression recherchée. D'une manière générale, les virus recombinants selon l'invention sont formulés et administrés sous forme de doses comprises entre 10⁴ et 10¹⁴ pfu, et de préférence 10⁶ à 10¹¹ pfu. Le terme pfu ("plaque forming unit") correspond au pouvoir infectieux du virus, et est déterminé par infection d'une culture cellulaire appropriée, et mesure du nombre de plages de cellules infectées. Les techniques de détermination du titre pfu d'une solution virale sont bien documentées dans la littérature.

En outre, les compositions selon l'invention peuvent également comprendre un agent de transfert chimique ou biochimique. On entend par le terme "agent de transfert chimique ou biochimique " tout composé (i.e., autre qu'un virus recombinant) facilitant la pénétration d'un acide nucléique dans une cellule. Il peut s'agir d'agents non viraux cationiques comme des lipides cationiques, des peptides, des polymères (Polyéthylène Imine, Polylysine), des nanoparticules ; ou d'agents non viraux non cationiques comme des liposomes non cationiques, des polymères ou des nanoparticules non cationiques.

Selon un mode préféré, les compositions selon l'invention comprennent un vecteur recombinant défectif comprenant un gène codant pour le transporteur de l'iode NIS et sont formulées pour une administration intratumorale. Avantageusement, les compositions de l'invention comprennent de 10⁴ et 10¹⁵ pfu, et de préférence 10⁷ à 10¹² pfu.

L'invention a également pour objet un procédé de préparation d'un médicament utile pour la prévention, l'amélioration et/ou le traitement des tumeurs caractérisé en ce que l'on mélange un vecteur recombinant comprenant un acide nucléique codant pour un transporteur de l'iode (NIS) avec un ou plusieurs adjuvants compatibles et pharmaceutiquement acceptables.

L'invention concerne également une méthode de traitement des tumeurs des mammifères, et notamment de l'homme, comprenant l'administration d'une quantité efficace d'un vecteur adénovirus recombinant défectif comprenant un acide nucléique codant pour le transporteur de l'iode (NIS).

Le terme « quantité efficace » désigne une quantité suffisante pour réduire d'au moins environ 15%, de préférence d'au moins 50 %, et de préférence encore d'au moins 90 % le volume des tumeurs, et plus préférentiellement encore une quantité suffisante pour l'élimination des tumeurs lorsque l'administration de l'adénovirus comprenant un acide nucléique codant pour le transporteur de l'iode (NIS) est associée à un traitement par radiothérapie métabolique à l'iode 135.

L'invention concerne le traitement des tumeurs et plus particulièrement le traitement des tumeurs solides. Parmi les tumeurs solides qui peuvent être traitées par l'objet de l'invention on peut citer notamment les sarcomes et les carcinomes, et à titre d'exemple non limitatif, les fibrosarcomes, les sarcomes ostéogéniques, les angiosarcomes, les endothéliosarcomes, les lymphangiosarcomes, les tumeurs de Ewing, le cancer du colon, le cancer du pancréas, le cancer des ovaires, le cancer de la prostate, les adénocarcinomes, les carcinomes du rein, du foie, du canal biliaire, les tumeur de Wilm's, le cancer du col de l'utérus, le cancer des testicules, le cancer du poumon, le cancer du poumon non à petite cellules, le cancer de la vessie, les carcinomes épithéliaux, les gliomes, les astrocytomes, les mélanomes, les neuroblastomes et les rétinoblastomes.

L'invention concerne également la prévention et/ou le traitement des désordres prolifératifs (telles que les métaplasies et les displasies) des tissus épithéliaux tels que l'épithélium du col de l'utérus, de l'oesophage et des poumons. A cet égard, l'invention concerne le traitement des conditions connues ou suspectées pour précéder une évolution vers une néoplasie ou un cancer, en particulier dans les états où la croissance de cellules non-néoplastiques telle que l'hyperplasie, la métaplasie, et plus particulièrement la displasie se produit (pour une revue de ces conditions anormales de croissance, voir Robbins and Angell. 1976, Basic Pathology, 2d Ed.. W.B. Saunders Co., Philadelphia, pp. 68-79). Hyperplasie est une forme de prolifération cellulaire contrôlée impliquant une augmentation du nombre de cellules dans un organe, sans altération structurale ou fonctionnelle significative de cet organe. Par exemple, une hyperplasie de l'endomètre peut précéder le cancer de l'endomètre. La métaplasie est une forme controlée de croissance cellulaire dans laquelle un type de cellule adulte ou totalement différenciée se substitue pour un autre type de cellule. La métaplasie peut se produire dans les tissus épithéliaux ou dans les tissus conjonctifs. La displasie est souvent un signe avant-coureur du cancer et se rencontre principalement au niveau de l'épithélium ; c'est la forme la plus fréquente de croissance cellulaire non-néoplastique impliquant la perte de l'uniformité des cellules individuelles et la perte de l'orientation structurelle des cellules. La displasie se produit de manière typique lorsqu'il existe une irritation ou une inflammation chroniques, et est souvent observée au niveau du col de l'utérus, les voies respiratoires , la cavité bucale, et sur la paroie de la vessie. Pour une revue, voir Fishman et al., 1985, Medicine, 2d Ed., J. B. Lippincott Co., Philadelphia.

La présente invention sera décrite plus en détail à l'aide des exemples qui suivent qui doivent être considérés comme illustratifs et non limitatifs.

### LEGENDE DES FIGURES

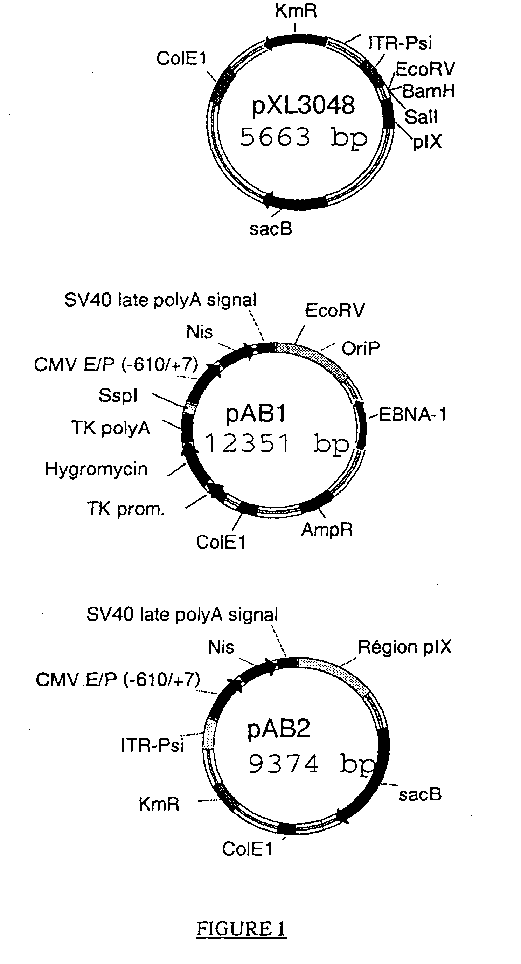

Figure 1 : schéma du plasmide pAB1, du plasmide pXL3048 et du plasmide pAB2. Le plasmide pXL3048 comprend l'extrémité gauche du génome de l'adénovirus de type 5 (nucléotides 1-382), un polylinker comprenant trois sites uniques de clonage et une partie du gène pIX (nucléotides 3446-4296). Le plasmide pAB2 résulte du clonage du fragment SspI-EcoRV de pAB1 dans pXL3048 préalablement linéarisé par EcoRV.

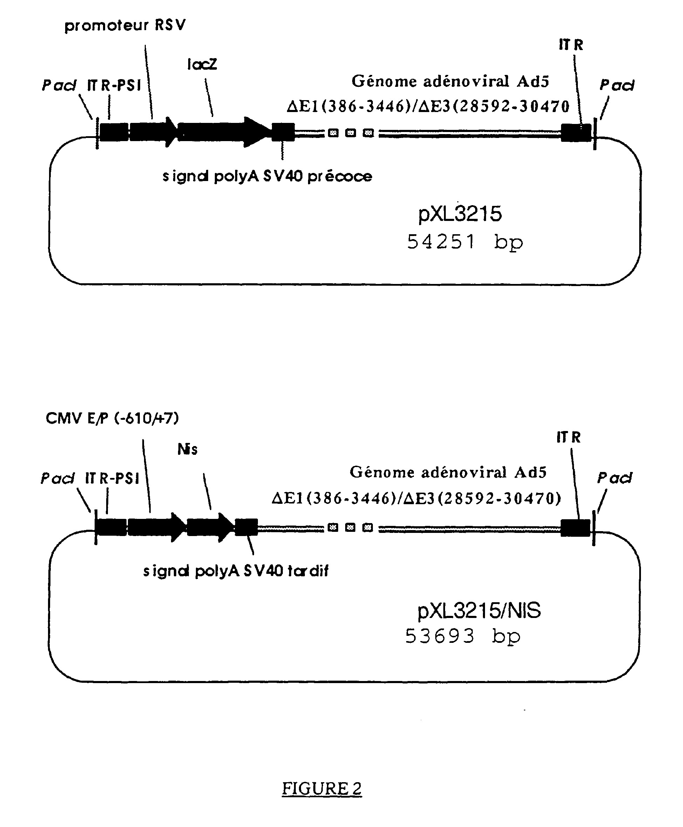

Figure 2 : obtention du plasmide pXL3215 généré par double recombinaison à partir des plasmides pAB2 et pXL3215 selon la méthode décrite par Crouzet et al. (PNAS vol 94 p1414, 1997). Le plasmide pXL3215 contient le génome d'un adénovirus de type 5 délété pour la région E 1 et E3 et contient la cassette d'expression de NIS.

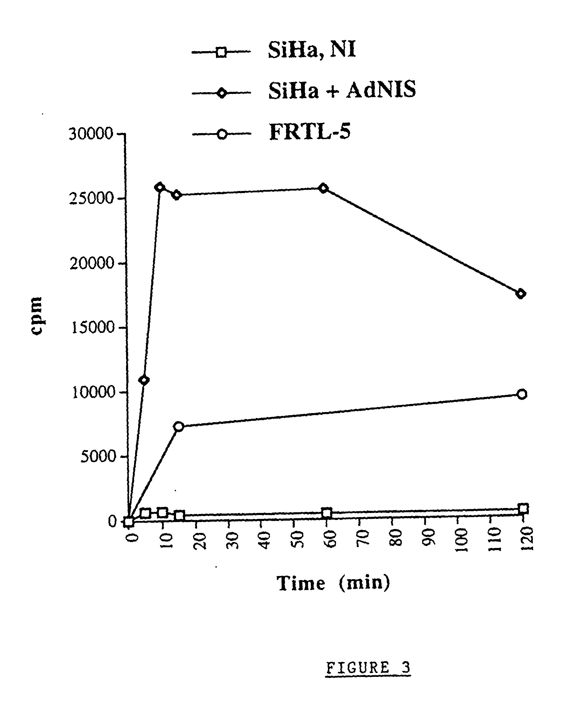

Figure 3 : cinétique d'accumulation de l'iode 125 par les cellules FTRL-5 et SiHa infectées avec le vecteur Ad-NIS (multiplicité d'infection 10). Les résultats de la cinétique sont exprimés en nombre de coups par minute pour 10⁶ cellules. La détermination du nombre de cellules par puits au moment du contact avec l'iode est la moyenne de deux mesures.

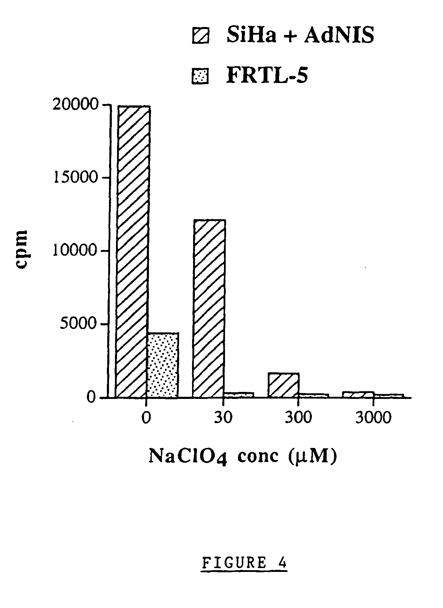

Figure 4 : inhibition spécifique du transport de l'iode par NIS en présence de perchlorate (NaClO₄) 30, 300 et 3000 µM. Le temps de contact des cellules avec l'iode 125 est de 15 minutes.

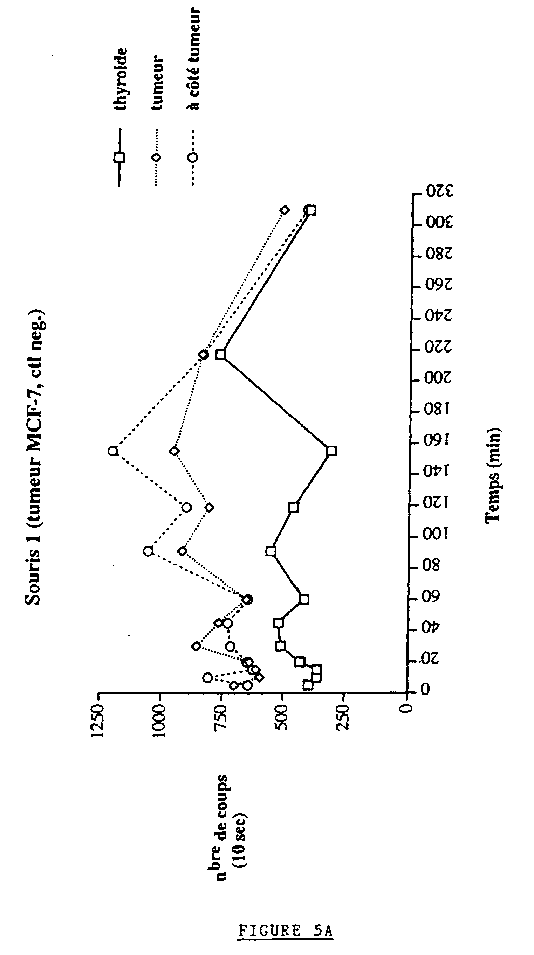

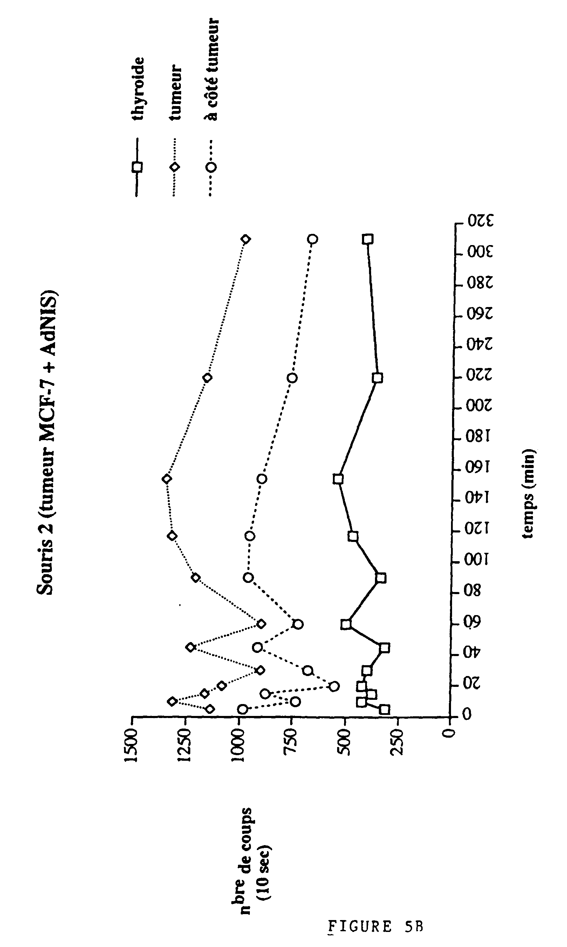

Figure 5 : accumulation de l'iode *in vivo* par des tumeurs infectées avec le vecteur Ad-NIS. Les cellules MCF-7 (cellules tumorales humaines de cancer mammaire) ont été injectées en sous-cutané chez la souris nude (5 x 10⁶ cellules). Après 15 jours (début d'apparition des tumeurs), des injections intrapéritonéales quotidiennes de thyroxine (2µg/animal/jour) ont été effectuées pendant 15 jours. Le vecteur Ad-CMV-NIS a ensuite été injecté à certains animaux en intratumoral (2 x 10⁹ pfu/tumeur ; taille des tumeurs 3-6mm). Trois jours après l'infection, 6µCi d'¹²⁵I sont injectés aux souris en intrapéritonéal. Les comptages sont effectués sur 10 secondes à intervalles de temps réguliers en plaçant la sonde sur la thyroide, sur la tumeur, et juste à proximité de la tumeur. Les résultats sont exprimés en nombre de coups détectés (mesure sur 10 secondes) en fonction du temps.

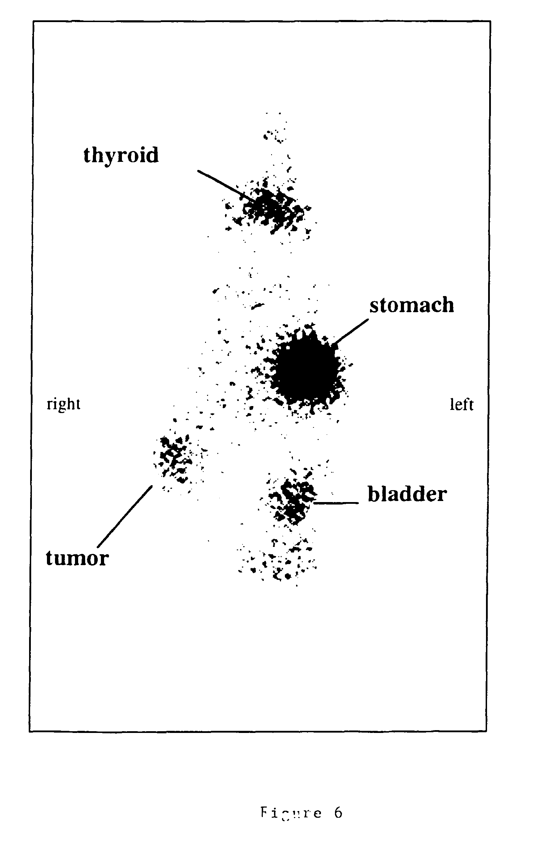

Figure 6 : scintigraphie d'une souris dont la tumeur a été infectée par le vecteur Ad-NIS. Les cellules MCF-7 (cellules tumorales humaines, cancer mammaire) ont été injectées en sous-cutané chez la souris nude (5 x 10⁶ cellules). Après 15 jours (début d'apparition des tumeurs), des injections intrapéritonéales quotidiennes de thyroxine (2µg/animal/jour) ont été effectuées pendant 15 jours. Le vecteur Ad-NIS a ensuite été injecté en intratumoral (2 x 10⁹ pfu/tumeur ; taille des tumeurs 3-6mm). Trois jours post-infection, 50 µCi d'¹²³I sont injectés en intrapéritonéal aux souris. Les comptages sont réalisés 1 heure après injection de l'iode. Le cliché est une vue ventrale de l'animal.

### MATERIEL ET METHODES

### Techniques générales de biologie moléculaire

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extractions de protéines au phénol ou au phénol-chloroforme, la précipitation d'ADN en milieu salin par de l'éthanol ou de l'isopropanol, la transformation dans *Escherichia coli,* etc ... sont bien connues de l'homme de métier et sont abondamment décrites dans la littérature [Maniatis T. et al., "Molecular Cloning, a Laboratory Manual", Cold Spring Harbor Laboratory, Cold Spring Harbor, N.Y., 1982; Ausubel F.M. et al. (eds). "Current Protocols in Molecular Biology", John Wiley & Sons, New York, 1987].

Pour les ligations, les fragments d'ADN peuvent être séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 (Biolabs) selon les recommandations du fournisseur.

Le remplissage des extrémités 5' proéminentes peut être effectué par le fragment de Klenow de l'ADN Polymérase 1 d'*E. coli* (Biolabs) selon les spécifications du fournisseur. La destruction des extrémités 3' proéminentes est effectuée en présence de l'ADN Polymérase du phage T4 (Biolabs) utilisée selon les recommandations du fabricant. La destruction des extrémités 5' proéminentes est effectuée par un traitement ménagé par la nucléase S1.

La mutagénèse dirigée in vitro par oligodéoxynucléotides synthétiques peut être effectuée selon la méthode développée par Taylor et al. [Nucleic Acids Res. 13 (1985) 8749-8764] en utilisant le kit distribué par Amersham.

L'amplification enzymatique de fragments d'ADN par la technique dite de PCR [Polymérase-catalyzed Chain Reaction. Saiki R.K. et al., Science 230 (1985) 1350-1354; Mullis K.B. et Faloona F.A., Meth. Enzym. 155 (1987) 335-350] peut être effectuée en utilisant un "DNA thermal cycler" (Perkin Elmer Cetus) selon les spécifications du fabricant.

La vérification des séquences nucléotidiques peut être effectuée par la méthode développée par Sanger et al. [Proc. Natl. Acad. Sci. USA, 74 (1977) 5463-5467] en utilisant le kit distribué par Amersham.

### EXEMPLES

### Exemple 1 : construction d'un adénovirus recombinant défectif exprimant le gène codant pour le transporteur spécifique de l'iode (Na⁺/I⁻ Symporteur) NIS.

Cet exemple décrit la construction d'un vecteur adénoviral défectif portant le gène codant pour le transporteur spécifique de l'iode (Na⁺/I⁻ Symporteur) NIS (ref Genbank U60282) lié de manière opérationnelle au promoteur CMV.

Le fragment comportant les nucléotides 74 à 2046 de la séquence du gène *nis* de rat (Dai et al., 1996, Nature 379 : 458-460) a été cloné sous forme d'un fragment AatII-HindIII aux sites PvuII-HindIII du plasmide pCEP-4 (Invitrogen). Le site AatII du fragment AatII-HindIII a préalablement été rendu bout franc par traitement à la nucléase Mung Bean (Biolabs) avant insertion dans le plasmide pCEP-4. Le plasmide résultant est nommé pAB 1.

Le plasmide pAB1 (figure 1) comprend le gène *nis* dont l'expression est placée sous contrôle de l'enhancer/promoteur du cytomégalovirus CMV (nucléotides -522 à +72) (Boshart et al. 1985. Cell, 41 : 521 - 530) et suivi du site de polyadénylation du virus SV40 (nucléotides 2538 à 2759 d'après génome de SV40, Genbank locus SV4CG).

L'ensemble formé par (i) l'enhancer/promoteur du cytomégalovirus , (ii) l'ADNc du gène *nis* et (iii), le site de polyadénylation du virus SV40 est appelé ci-après la cassette d'expression de NIS.

Le fragment SspI-EcoRV (3711 bp) du plasmide pAB 1 comprenant la cassette d'expression de NIS définie ci-dessus est ensuite cloné dans le vecteur navette pXL3048 préalablement linéarisé par EcoRV. Le vecteur résultant est nommé pAB2 (figure 2). Le plasmide pXL3048 est un plasmide dérivé du plasmide Kan-SacB (Crouzet et al. PNAS vol 94 p1414-1419, 1997) comprenant l'extrémité gauche du génome de l'adénovirus de type 5 (nucléotides 1-382), un polylinker comprenant trois sites uniques de clonage et une partie du gène pIX (nucléotides 3446-4296).

L'adénovirus Ad-NIS a été construit selon la méthode décrite par Crouzet et al. (PNAS vol 94 p1414-1419, 1997) par recombinaison homologue entre le plasmide pAB2 et le plasmide pXL3215.

Le plasmide pXL3215 contient le génome d'un adénovirus contenant une cassette RSV-LacZ insérée dans sa région E1 (ΔE 1 386-3446) et une délétion dans la région E3 (ΔE3 28592-30470).

Le principe de la construction est décrit dans la figure 2. Le plasmide pXL3215généré par cette double recombinaison contient le génome d'un adénovirus de type 5 délété pour la région E1 et E3 et contenant la cassette d'expression CMV-NIS-poly A SV40. Cette construction est vérifiée par séquençage de la cassette d'expression de NIS.

L'adénovirus Ad-NIS est généré par infection de l'ADN de pXL3215 digéré par PacI dans la lignée 293 (ATCC CRL-1573). Les particules virales obtenues sont ensuite amplifiées dans cette même lignée et des stocks de virus produits sont purifiés par double gradient de CsCl.

Les particules virales sont ensuite utilisées pour étudier l'expression du gène NIS dans différentes lignées tumorales.

### Exemple 2 : accumulation de l'iode in vitro par des cellules tumorales infectées avec le vecteur Ad-NIS

La capacité d'accumulation de l'iode dans les cellules infectées avec le vecteur Ad-NIS a été testée dans différentes lignées: des cellules tumorales épithéliales SiHa (ATCC HTB-35), des cellules tumorales mammaires MCF7 (ATC HTB-22) et T-47D (ATCC HTB-133) et des cellules tumorales d'origine prostatique DU-145 (ATCC HTB-81) et PC-3 (ATCC CRL-1435).

Les cellules sont infectées avec le vecteur Ad-NIS à une multiplicité d'infection de 10. La capacité des cellules à capter l'iode est testée 28 à 29h après infection selon la méthode de Weiss et al. [Endocrinology (1984) 114 :1090-1098]. Les cellules infectées sont lavées avec du tampon HBSS et mises en contact pendant le temps indiqué dans la figure 3 avec 0,5ml de tampon HBSS contenant 0,1 µCi d'iode 125. A la fin du temps de contact, les cellules sont lavées une fois avec du tampon HBSS froid, puis incubées pendant 20 min avec de l'éthanol froid. La quantité d'iode 125 captée par les cellules est déterminée par dosage de la radioactivité de l'éthanol, avec un compteur gamma.

Les résultats obtenus avec la lignée SiHa sont présentés dans la figure 3 et peuvent être comparés à ceux obtenus avec les cellules FRTL-5 (cellules thyroidiennes de rat, captant l'iode naturellement et servant de contrôle).

L'inhibition spécifique du transport de l'iode par NIS a été testée en présence de perchlorate. Le NaClO₄ est ajouté dans le tampon HBSS contenant l'iode 125 de façon à obtenir des concentrations finales de 30, 300 et 3000 µM. Le temps de contact des cellules avec l'iode 125 est de 15 minutes. Les résultats sont présentés dans la figure 4. Les résultats sont exprimés en nombre de coups pour un même nombre de cellules.

Les cellules FRTL-5 (contrôle positif) fixent une certaine quantité d'iode, et on observe que ce phénomène est très rapide (maximum presque atteint après 15 min de contact). Les résultats obtenus montrent que les cellules SiHa (cellules épithéliales humaines, tumorales) non infectées ne captent pas d'iode. De manière surprenante, on observe que les cellules SiHa infectées avec le vecteur Ad-NIS captent jusqu'à 5 fois plus d'iode que les cellules FRTL-5. On remarque également que la cinétique initiale du phénomène est comparable à celle observée pour les cellules FRTL-5. La spécificité du transfert a été confirmée par infection avec un adénovirus contrôle ne comportant pas le gène *nis* (résultat non présenté).

Les résultats des expériences d'inhibition (figure 4) montrent que la fixation d'iode dans les cellules FRTL-5 est inhibée à plus de 90% à partir d'une concentration en NaClO₄ de 30µM (93% pour 30 µM, 95% aux concentrations supérieures). Il est intéressant de noter que, par contre, pour les cellules SiHa infectées par Ad-NIS, la concentration en inhibiteur nécessaire pour obtenir le même taux d'inhibition est beaucoup plus élevée (40% d'inhibition seulement pour 30µM en NaClO₄, 92% d'inhibition pour 300µM en NaClO₄ et 98% pour 3 mM en NaClO₄).

L'expérience d'inhibition permet de conclure que la capacité des cellules SiHa infectées par Ad-NIS à capter l'iode est bien due à l'expression de NIS dans ces cellules, puisque le phénomène est inhibé par NaCIO, qui est un inhibiteur compétitif et spécifique du transport des ions iodures par NIS. Des résultats similaires ont étés obtenus pour d'autres lignées cellulaires tumorales humaines (cellules tumorales mammaires : MCF-7 et T47-D, et cellules tumorales d'origine prostatique : DU-145 et PC-3).

Les résultats concernant l'inhibition par NaClO₄ confirment également que l'infection de cellules tumorales par Ad-NIS conduit à un niveau d'expression de NIS particulièrement élevé, puisque des concentrations élevées en NaClO₄ sont requises pour inhiber le phénomène (dans le cas des cellules SiHa, et par rapport aux cellules FRTL-5, une concentration en inhibiteur 10 fois supérieure est requise pour obtenir un même taux d'inhibition).

L'ensemble de ces résultats démontre que le vecteur adénoviral est un vecteur particulièrement avantageux pour le transfert du gène *nis* et que l'utilisation d'un tel vecteur permet d'atteindre un niveau d'accumulation de l'iode dans des cellules tumorales non thyroïdiennes de l'ordre de 5 fois supérieur à celui observé pour les cellules captant naturellement l'iode 125.

### Exemple 3 : accumulation de l'iode in vivo par des tumeurs infectées par le vecteur Ad-NIS

Des cellules MCF-7 (cellules tumorales humaines de cancer mammaire) ont été injectées en sous-cutané chez la souris nude (5 x 10⁶ cellules). Après 15 jours (début d'apparition des tumeurs), des injections intrapéritonéales quotidiennes de thyroxine (2µg/animal/jour) ont été effectuées pendant 15 jours, de façon à mettre la thyroide des animaux au repos. Le vecteur Ad-NIS a ensuite été injecté à certains animaux en intratumoral (2 x 10° pfu/tumeur) ; le diamètre des tumeurs au moment de l'injection était de 3 à 6 mm. Trois jours après l'infection, 6 µCi d'¹²⁵I ont été injectés en intrapéritonéal aux souris, et des comptages sur 10 secondes ont été effectués à l'aide d'une sonde pouvant être déplacée à différents endroits de l'animal. Des comptages ont été effectués à intervalles de temps réguliers en plaçant la sonde sur la thyroide, sur la tumeur, et juste à proximité de la tumeur. Les résultats sont présentés sur la figure 5. Les résultats sont exprimés en nombre de coups détectés (mesure sur 10 secondes) en fonction du temps.

Dans les animaux contrôles (sans administration de Ad-NIS), la thyroide fixe une petite quantité d'iode, mais grâce au traitement par la thyroxine, on n'observe pas d'accumulation importante de l'iode dans la thyroide au cours du temps. On n'observe pas de fixation d'iode dans la tumeur dans les animaux contrôles (voir figure 5A). Le fait que NIS soit naturellement présent dans l'estomac, ainsi que le fait que l'iode soit éliminé par voie urinaire expliquent les valeurs relativement élevées observées (bruit de fond relativement important).

Les animaux traités avec Ad-NIS administré en intratumoral (figure 5B) ne présentent pas d'accumulation importante d'iode au niveau de la thyroïde (effet du traitement par la thyroxine). Sur ces animaux, les quantités d'iode détectées en plaçant la sonde sur la tumeur sont systématiquement plus élevées que les quantité d'iode détectées à proximité de la tumeur, ce qui indique qu'il y a bien fixation de l'iode dans la tumeur après traitement par le vecteur Ad-NIS.

Afin de confirmer la fixation de l'iode au niveau des tumeurs, des mesures de radioactivité par scintigraphie ont été réalisées sur les animaux traités par le vecteur Ad-NIS. Les résultats obtenus sont présentés sur la figure 6. Les animaux utilisés ont été préparés selon le protocole décrit ci-dessus, sauf que 50 µCi d'¹²³I ont été injectés en intrapéritonéal ; l'image a été obtenue une heure après injection de l'iode.

Le cliché est une vue ventrale de l'animal. Quatre zones de fixation de l'iode sont visibles : la thyroide, la vessie (voie d'élimination de l'iode), l'estomac (dans lequel NIS est exprimé naturellement), et la tumeur. Ces résultats de scintigraphie confirment les résultats précédents obtenus par comptage et montrent que les vecteurs selon l'invention permettent d'obtenir une fixation importante de l'iode au niveau des tumeurs.

### Exemple 4 : évaluation quantitative de l'accumulation de l'iode in vivo par des tumeurs infectées par le vecteur Ad-NIS

Des cellules SiHa (5 x 10⁶ cellules) ont été injectées en sous-cutané chez la souris nude (n=12) sur les deux flancs de l'animal. Après 15 jours (début d'apparition des tumeurs), des injections intrapéritonéales quotidiennes de thyroxine (2µg/animal/jour) ont été effectuées pendant 15 jours, de façon à mettre la thyroide des animaux au repos. Pour chaque animal, le vecteur Ad-NIS a ensuite été injecté en intratumoral (2 x 10⁹ pfu/tumeur) sur une tumeur ; l'autre tumeur servant de contrôle ; le diamètre des tumeurs au moment de l'injection était de 5 à 8 mm. Trois jours après l'infection, 6 µCi d'¹²⁵I ont été injectés en intrapéritonéal aux souris. Les animaux ont été sacrifiés 90 minutes après l'administration d'¹²⁵I. et la quantité d'¹²⁵I a été déterminée. Les résultats sont présentés dans le tableau 1. Les résultats sont exprimés en pourcentage d'¹²⁵Iadministré par gramme de tissu.

| souris | Tumeur traitée avec Ad-NIS | Tumeur contrôle | ratio |
|---|---|---|---|
| | (%¹²⁵I administré / g de tissu) | (%¹²⁵I administré/ g de tissu) | |
| 1 | 9.53 | 1.75 | 5.4 |
| 2 | 16.81 | 0.88 | 19.1 |
| 3 | 5.56 | 1.08 | 5.1 |
| 4 | 11.93 | 1.51 | 7.9 |
| 5 | 4.26 | 1.02 | 4.2 |
| 6 | 12.91 | 0.92 | 14.0 |
| 7 | 12.76 | 1.16 | 11.0 |
| 8 | 5.53 | 1.42 | 3.9 |
| 9 | 10.75 | 1.45 | 7.4 |
| 10 | 14.52 | 0.85 | 17.1 |
| 11 | 10.10 | 0.41 | 24.6 |
| 12 | 10.91 | 1.17 | 9.3 |
| | 10.46 ± 3.79 | 1.14 ± 0.36 | 10.8 ± 6.6 |

Les résultats obtenus montrent que les tumeurs traitées avec le vecteur Ad-NIS sont capables d'accumuler l'iode ¹²⁵I de manière très efficace et jusqu'à 25 fois plus que dans les tumeurs non traitées. Ces résultats confirment les résultats précédents et montrent que les vecteurs selon l'invention permettent d'obtenir une fixation importante de l'iode au niveau des tumeurs.

## Revendications

1. Adénovirus recombinant défectif **caractérisé en ce qu'**il comprend au moins une séquence d'ADN codant pour le transporteur spécifique de l'iode (Na⁺/I⁻ Symporteur) NIS ou un dérivé de celui-ci conservant une activité de transport spécificique de l'iode, ladite séquence d'ADN étant sous le contrôle d'un promoteur transcriptionnel permettant son expression.

2. Adénovirus selon la revendication 1 **caractérisé en ce que** la séquence d'ADN est une séquence d'ADNc.

3. Adénovirus selon la revendication 1 **caractérisé en ce que** la séquence d'ADN est une séquence d'ADNg.

4. Adénovirus selon l'une des revendications 1 à 3 **caractérisé en ce que** la séquence d'ADN code pour le transporteur spécifique de l'iode (Na⁺/I⁻ Symporteur) NIS murin.

5. Adénovirus selon l'une des revendications 1 à 3 **caractérisé en ce que** la séquence d'ADN code pour le transporteur spécifique de l'iode (Na⁺/I⁻ Symporteur) NIS humain.

6. Adénovirus selon l'une des revendications 1 à 5, **caractérisé en ce que** la séquence d'ADN est placée sous le contrôle d'un promoteur transcriptionnel permettant son expression spécifique dans les cellules tumorales.

7. Adénovirus selon la revendication 6 **caractérisé, en ce que** le promoteur transcriptionnel est le promoteur MT-1 ou est choisi parmi les promoteurs viraux, de préférence parmi les promoteurs E1A, MLP, CMV et LTR-RSV, SV40.

8. Adénovirus recombinant défectif comprenant une séquence d'ADNc codant pour le transporteur de l'iode NIS humain sous le contrôle du promoteur CMV.

9. Adénovirus recombinant défectif comprenant une séquence d'ADN codant pour le transporteur de l'iode NIS ou un dérivé de celui-ci conservant une activité de transport spécifique de l'iode, ladite séquence d'ADN étant sous le contrôle d'un promoteur permettant une expression majoritaire dans les cellules tumorales.

10. Adénovirus recombinant défectif selon la revendication 9, **caractérisé en ce que** le promoteur est choisi parmi la séquence de régulation du gène de l'élastase I , du gène de l'insuline, des gènes des immunoglobulines, du virus des tumeurs mammaires de souris, du gène PSA, du gène de l'alpha-foetopmtéine, du gène de l'alpha 1-antitrypsine, du gène de la β-globine, du gène de la myéline basique, du gène de la chaîne légère 2 de la myosine et du gène de l'hormone de libération des gonadotrophines.

11. Adénovirus selon l'une des revendications 1 à 10, **caractérisé en ce qu'**il ce qu'il comprend au moins une délétion de tout ou partie de la région E1 et une délétion de tout ou partie de la région E4.

12. Adénovirus selon la revendication 11, **caractérisé en ce qu'**il comprend en outre une délétion de tout ou partie de la région E3.

13. Adénovirus selon l'une des revendications 1 à 12, **caractérisé en ce qu'**il s'agit d'un adénovirus humain de type Ad 2 ou Ad 5 ou canin de type CAV-2.

14. Adénovirus selon l'une des revendications 1 à 13, **caractérisé en ce qu'**il comporte en outre au moins un gène codant pour un polypeptide impliqué dans l'organification de l'iode, de préférence un gène codant pour un polypeptide impliqué dans un système peroxydasique, tel que le gène de la glucose oxidase.

15. Utilisation d'un adénovirus selon l'une des revendications 1 à 14 pour la préparation d'une composition pharmaceutique destinée au traitement et/ou à l'inhibition de la croissance des tumeurs.

16. Composition pharmaceutique comprenant un ou plusieurs adénovirus recombinants défectifs selon l'une des revendications 1 à 14.

17. Composition pharmaceutique selon la revendication 16 **caractérisée en ce qu'**elle est sous forme injectable.

18. Composition pharmaceutique selon la revendication 16 ou 17, **caractérisée en ce qu'**elle comprend entre 10⁴ et 1014 pfu/ml, et de préférence 10⁶ à 10¹¹ pfu/ml adénovirus recombinants défectifs.

## Claims

1. Defective recombinant adenovirus, **characterized in that** it comprises at least one DNA sequence encoding the specific iodine transporter (Na⁺/I⁻ Symporter) NIS or a derivative thereof preserving an iodine specific transport activity, the said DNA sequence being under the control of a transcriptional promoter allowing its expression.

2. Adenovirus according to Claim 1, **characterized in that** the DNA sequence is a cDNA sequence.

3. Adenovirus according to Claim 1, **characterized in that** the DNA sequence is a gDNA sequence.

4. Adenovirus according to one of Claims 1 to 3, **characterized in that** the DNA sequence encodes the specific murine iodine transporter (Na⁺/I⁻ Symporter) NIS.

5. Adenovirus according to one of Claims 1 to 3, **characterized in that** the DNA sequence encodes the specific human iodine transporter (Na⁺/I⁻ Symporter) NIS.

6. Adenovirus according to one of Claims 1 to 5, **characterized in that** the DNA sequence is placed under the control of a transcriptional promoter allowing its specific expression in tumor cells.

7. Adenovirus according to Claim 6, **characterized in that** the transcriptional promoter is the MT-1 promoter or is chosen from viral promoters, preferably from the promoters E1A, MLP, CMV and RSV-LTR, SV40.

8. Defective recombinant adenovirus comprising a cDNA sequence encoding the human iodine transporter NIS under the control of the CMV promoter.

9. Defective recombinant adenovirus comprising a DNA sequence encoding the iodine transporter NIS or a derivative thereof preserving an iodine specific transport activity, the said DNA sequence being under the control of a promoter allowing predominant expression in tumor cells.

10. Defective recombinant adenovirus according to Claim 9, **characterized in that** the promoter is chosen from the regulatory sequence of the elastase I gene, the insulin gene, the gene for immunoglobulins, the mouse mammary tumor virus, the PSA gene, the alpha-fetoprotein gene, the alpha 1-antitrypsin gene, the β-globin gene, the gene for basic myelin, the gene for the myosin light chain 2 and the gene for the gonadotrophin-releasing hormone.

11. Adenovirus according to one of Claims 1 to 10, **characterized in that** it that it comprises at least a deletion of all or part of the E1 region and a deletion of all or part of the E4 region.

12. Adenovirus according to Claim 11, **characterized in that** it comprises, in addition, a deletion of all or part of the E3 region.

13. Adenovirus according to one of Claims 1 to 12, **characterized in that** it is a human adenovirus type Ad 2 or Ad 5 or a canine adenovirus type CAV-2.

14. Adenovirus according to one of Claims 1 to 13, **characterized in that** it comprises, in addition, at least one gene encoding a polypeptide involved in the organification of iodine, preferably a gene encoding a polypeptide involved in a peroxidase system such as the gene for glucose oxidase.

15. Use of an adenovirus according to one of Claims 1 to 14, for the preparation of a pharmaceutical composition intended for treating and/or for inhibiting the growth of tumors.

16. Pharmaceutical composition comprising one or more defective recombinant adenoviruses according to one of Claims 1 to 14.

17. Pharmaceutical composition according to Claim 16, **characterized in that** it is in injectable form.

18. Pharmaceutical composition according to Claim 16 or 17, **characterized in that** it comprises between 10⁴ and 10¹⁴ pfu/ml, and preferably 10⁶ to 10¹¹ pfu/ml defective recombinant adenoviruses.

## Patentansprüche

1. Defektives rekombinantes Adenovirus, **dadurch gekennzeichnet, daß** es mindestens eine DNA-Sequenz, die für den spezifischen Iodtransporter NIS (Na⁺/I⁻-Symporter) oder ein Derivat davon, bei dem eine spezifische Iodtransportaktivität erhalten bleibt, codiert, wobei diese DNA-Sequenz unter der Kontrolle eines Transkriptionspromoters steht, der deren Expression gestattet.

2. Adenovirus nach Anspruch 1, **dadurch gekennzeichnet, daß** es sich bei der DNA-Sequenz um eine cDNA-Sequenz handelt.

3. Adenovirus nach Anspruch 1, **dadurch gekennzeichnet, daß** es sich bei der DNA-Sequenz um eine gDNA-Sequenz handelt.

4. Adenovirus nach einem der Ansprüche 1 bis 3, **dadurch gekennzeichnet, daß** die DNA-Sequenz für den spezifischen Iodtransporter NIS (Na⁺/I⁻-Symporter) der Maus codiert.

5. Adenovirus nach einem der Ansprüche 1 bis 3, **dadurch gekennzeichnet, daß** die DNA-Sequenz für den spezifischen Iodtransporter NIS (Na⁺/I⁻-Symporter) des Menschen codiert.

6. Adenovirus nach einem der Ansprüche 1 bis 5, **dadurch gekennzeichnet, daß** die DNA-Sequenz unter die Kontrolle eines Transkriptionspromoters gestellt wird, der ihre spezifische Expression in Tumorzellen gestattet.

7. Adenovirus nach Anspruch 6, **dadurch gekennzeichnet, daß** es sich bei dem Transkriptionspromoter um den MT-1-Promoter handelt oder daß er aus der Reihe der viralen Promoter, insbesondere aus der Reihe der Promoter E1A, MLP, CMV und LTR-RSV, SV40, stammt.

8. Defektives rekombinantes Adenovirus, umfassend eine cDNA-Sequenz, die für den menschlichen NIS-Iodtransporter unter der Kontrolle des CMV-Promoters codiert.

9. Defektives rekombinantes Adenovirus, umfassend eine DNA-Sequenz, die für den NIS-Iodtransporter oder ein Derivat davon, bei dem eines spezifische Iodtransportaktivität beibehalten ist, codiert, wobei diese DNA-Sequenz unter der Kontrolle eines Promoters steht, der eine Expression hauptsächlich in den Tumorzellen gestattet.

10. Defektives rekombinantes Adenovirus nach Anspruch 9, **dadurch gekennzeichnet, daß** der Promoter aus der Reihe Regulationssequenz des Elastase-I-Gens, des Insulingens, der Immunglobulingene, des Mamma-Tumor-Virus der Maus, des PSA-Gens, des Alpha-Fötalprotein-Gens, des Alpha-1-Antitrypsin-Gens, des Beta-Globingens, des basischen Myelingens, des Gens für die Myosin-Leichtkette 2 und des Gonadoliberin-Gens stammt.

11. Adenovirus, nach einem der Ansprüche 1 bis 10, **dadurch gekennzeichnet, daß** es mindestens eine Deletion der gesamtem Region E1 oder eines Teils davon sowie eine Deletion der gesamten Region E4 oder eines Teils davon umfaßt.

12. Adenovirus nach Anspruch 11, **dadurch gekennzeichnet, daß** es weiterhin eine Deletion der gesamten Region E3 oder eines Teil davon umfaßt.

13. Adenovirus nach einem der Ansprüche 1 bis 12, **dadurch gekennzeichnet, daß** es sich um ein menschliches Adenovirus des Typs AD2 oder AD5 oder ein Hunde-Adenovirus des Typs CAV-2 handelt.

14. Adenovirus nach einem der Ansprüche 1 bis 13, **dadurch gekennzeichnet, daß** es weiterhin mindestens ein Gen umfaßt, das für ein Polypeptid codiert, welches am Einbau von Iod in die Organe beteiligt ist, vorzugsweise ein Gen, das für ein Polypeptid codiert, das an einem Peroxydasesystem beteiligt ist, wie dem Glucoseoxydasegen.

15. Verwendung eines Adenovirus nach einem der Ansprüche 1 bis 14 zur Herstellung einer pharmazeutischen Zusammensetzung zur Behandlung und/oder Hemmung des Wachstums von Tumoren.

16. Pharmazeutische Zusammensetzung, die ein oder mehrere defektive rekombinante Adenoviren nach einem der Ansprüche 1 bis 14 enthalten.

17. Pharmazeutische Zusammensetzung nach Anspruch 16, **dadurch gekennzeichnet, daß** sie in injizierbarer Form vorliegt.

18. Pharmazeutische Zusammensetzung nach Anspruch 16 oder 17, **dadurch gekennzeichnet, daß** sie zwischen 10⁴ und 10¹⁴ pfu/ml, vorzugsweise 10⁶ bis 10¹¹ pfu/ml, defektives rekombinantes Adenovirus enthält.
